(19)

**Europäisches Patentamt**
**European Patent Office**
**Office européen des brevets**

(11) **EP 4 257 956 A1**

(12) **EUROPEAN PATENT APPLICATION**
published in accordance with Art. 153(4) EPC

(43) Date of publication:
**11.10.2023 Bulletin 2023/41**

(51) International Patent Classification (IPC):
**G01N 21/64** (2006.01)

(21) Application number: **21956558.7**

(52) Cooperative Patent Classification (CPC):
**Y02A 20/20**

(22) Date of filing: **03.11.2021**

(86) International application number:
**PCT/CN2021/128294**

(87) International publication number:
**WO 2023/035386 (16.03.2023 Gazette 2023/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.09.2021 CN 202111063004**

(71) Applicant: **INSTITUTE OF OCEANOGRAPHIC
INSTRUMENTATION,
SHANDONG ACADEMY OF SCIENCES**
**Qingdao, Shandong 266000 (CN)**

(72) Inventors:
 • **ZHANG, Yingying**
  **Qingdao, Shandong 266000 (CN)**
 • **GAO, Shun**
  **Qingdao, Shandong 266000 (CN)**
 • **YUAN, Da**
  **Qingdao, Shandong 266000 (CN)**
 • **ZHANG, Yunyan**
  **Qingdao, Shandong 266000 (CN)**
 • **WU, Bingwei**
  **Qingdao, Shandong 266000 (CN)**
 • **FENG, Xiandong**
  **Qingdao, Shandong 266000 (CN)**

(74) Representative: **Cabinet Beau de Loménie
158, rue de l'Université
75340 Paris Cedex 07 (FR)**

(54) **METHOD AND APPARATUS FOR CALIBRATING OPTICAL DISSOLVED OXYGEN SENSOR**

(57) The present invention discloses a calibration method and a calibration apparatus for an optical dissolved oxygen sensor, wherein the apparatus comprises a sealed cylinder for accommodating the optical dissolved oxygen sensor, a constant temperature water tank for regulating the temperature inside the cylinder body, an air bottle and a nitrogen bottle for filling air into the sealed cylinder, a sensor module for detecting temperature, pressure and humidity in the cylinder body and a data acquisition and control module, which provides a hardware support for in-situ measurement and calibration of dissolved oxygen value of a measured medium; the method comprises two phases of setting an error model and calibrating, not only could determine if the optical dissolved oxygen sensor has a problem of data drift, but also able to perform automatic calibration of the dissolved oxygen concentration detected by the optical dissolved oxygen sensor, thereby achieving an accurate acquisition of the dissolved oxygen concentration of a measured media.

S21 — Obtaining a measured dissolved oxygen concentration of a measured medium by an optical dissolved oxygen sensor in practical application

S22 — Placing the optical dissolved oxygen sensor in practical application in a sealed gaseous environment filled with air

S23 — Collecting an ambient temperature of a place where the optical dissolved oxygen sensor in practical application is located and a dissolved oxygen concentration detected by the optical dissolved oxygen sensor in practical application

S24 — Determining if data drift occurs by the error model

No data drift occurs

Data drift occurs

Using the measured dissolved oxygen concentration as a final result

S25 — Placing the optical dissolved oxygen sensor in practical application is a sealed gaseous oxygen-free environment and obtaining a dissolved oxygen concentration detected by the optical dissolved oxygen sensor in practical application

S26 — Using the calibration model to calibrate the measured value of dissolved concentration and obtaining a calibration value of dissolved oxygen concentration

Fig.3

EP 4 257 956 A1

**Description**

Technical Field

The present invention belongs to the technical field of instrument calibration and relates in particular to a method and an apparatus for calibrating an optical dissolved oxygen sensor in which data drift occurs.

Background

Dissolved oxygen is an important indicator used to assess seawater quality and water body contamination, and could even be considered as one of the most important seawater monitoring indicators, along with temperature and salinity. The level of dissolved oxygen in seawater has a major impact on the activities of marine life and marine aquaculture, and is an important basis for marine environmental assessment and marine science experiments.

The methods used to monitor the dissolved oxygen level in seawater include the iodometric method, the membrane electrode method and the measurement method using optical dissolved oxygen sensor is used. The optical dissolved oxygen sensor, based on the principle of fluorescence quenching, has become internationally accepted as the most discussed measurement method for automatic dissolved oxygen monitoring in water due to its fast response time, low oxygen consumption in seawater, long lifetime and low environmental impact.

Optical dissolved oxygen sensors based on fluorescence quenching are suitable for long-term in-situ monitoring of dissolved oxygen, but during the storage period from calibration to delivery and in practical use, "storage drift" and "measurement drift" inevitably occur, leading to a significant reduction in the accuracy and reliability of the measurement data. It is therefore necessary to calibrate sensors where data drift occurs. However, the usual calibration method is mainly carried out in the laboratory and is very time-consuming and costly, as the dissolved oxygen sensor has to be transported from its original location to the laboratory. Hence, it is very important to explore the in-situ self-calibration method for dissolved oxygen sensors to ensure the reliability and stability of in-situ dissolved oxygen monitoring, which will play an important role in improving the data quality of in-situ dissolved oxygen sensor monitoring and extending the maintenance-free operation time.

Summary

It is an object of the present invention to provide a method for calibrating an optical dissolved oxygen sensor, which aims to solve the problem of calibrating the optical dissolved oxygen sensor after the occurrence of data drift.

To solve the above technical problems, the following technical solutions are implemented in the present invention.

In one aspect, the present invention discloses an optical dissolved oxygen sensor calibration method comprising two phases.

Firstly, generating an error model with an accurate optical dissolved oxygen sensor, which includes:

placing a standard optical dissolved oxygen sensor in an air-filled sealed gaseous environment;

under each of different temperatures $t$, obtaining an oxygen partial pressure value $O_2^{sensor}(t)$ corresponding to a concentration of dissolved oxygen detected by the standard optical dissolved oxygen sensor and a theoretical partial pressure value of oxygen $O_2^{air}(t)$ of oxygen in the air-filled sealed gaseous environment; and

establishing an error model: $Y_1(t) = At^2 + Bt + C$, wherein $A, B,$ and $C$ are coefficients and determined by

$$Y_1(t) = \frac{O_2^{sensor}(t) - O_2^{air}(t)}{O_2^{air}(t)}\%$$ and the temperature $t$;

secondly, calibrating a measured dissolved oxygen concentration $O_2^{sea,sensor}$ of a measured medium detected by an optical dissolved oxygen sensor in practical measurement by a calibration model, which includes:

placing the optical dissolved oxygen sensor in practical measurement in an air-filled sealed gaseous environment to obtain a dissolved oxygen concentration $O_2^{100\%air,sensor}$ detected by the optical dissolved oxygen sensor in practical measurement;

placing the optical dissolved oxygen sensor in practical measurement in an oxygen-free sealed gaseous environment and obtaining a dissolved oxygen concentration $O_2^{0\%air,sensor}$ detected by the optical dissolved oxygen sensor in practical measurement; and

using a calibration model to calibrate the measured dissolved oxygen concentration $O_2^{sea,sensor}$ to obtain a calibration value of dissolved oxygen concentration $O_2^{corr}$; in which the calibration model is:

$$O_2^{corr} = D + E \times O_2^{sea,sensor},$$

in which,

$$D = -E \times O_2^{0\%air,sensor};$$

$$E = Y_2 / \left( O_2^{100\%air,sensor} - O_2^{0\%air,sensor} \right);$$

$$Y_2 = \left(1 - \frac{Y_1}{100}\right) O_2^{100\%air,sensor};$$

$$Y_1 = Y_1(t0) = At0^2 + Bt0 + C;$$

Wherein $t0$ is the temperature of the measured medium.

In another aspect, the present invention also discloses an optical dissolved oxygen sensor calibration apparatus comprising:

a sealed cylinder including a cylinder body provided with a water inlet, a water outlet, gas inlets and an gas outlet, which is configured to accommodate an optical dissolved oxygen sensor;

a constant temperature water tank containing liquids flooding the sealed cylinder;

an air bottle connected to one gas inlet of the sealed cylinder via an air pipe, which is configured to inject saturated air into the cylinder body of the sealed cylinder;

a nitrogen bottle connected to the other gas inlet of the sealed cylinder via a nitrogen pipe, which is configured to inject nitrogen into the cylinder body of the sealed cylinder;

a sensor module disposed in the cylinder body of the sealed cylinder, which is configured to detect the temperature, pressure and humidity inside the cylinder body; and

a data acquisition and control unit configured to execute a data acquisition and control process including:

controlling the oxygen bottle to inject air into the sealed cylinder to fill the cylinder body with saturated air;

collecting a temperature inside the cylinder body t0 detected by the sensor module and a dissolved oxygen concentration $O_2^{100\%air,sensor}$ detected by the optical dissolved oxygen sensor in practical measurement accommodated in the cylinder body; calculating an oxygen partial pressure value $O_2^{sensor}(t)$ corresponding to the dissolved oxygen concentration;

calculating $Y_1(t)$, wherein $Y_1 = Y_1(t0) = At0^2 + Bt0 + C$; wherein $A$, $B$, and $C$ are constant coefficients;

calculating a difference between $Y_1$ and $\dfrac{O_2^{sensor}(t0) - O_2^{air}(t0)}{O_2^{air}(t0)}\%$ ;

determining that the optical dissolved oxygen sensor in practical measurement has data drift if the difference is beyond a set error range; wherein $O_2^{air}(t0)$ is a theoretical partial pressure value of oxygen in the saturated air in the sealed cylinder under the temperature $t0$;

opening the water inlet and the gas outlet of the sealed cylinder, injecting and filling the cylinder body with a water body to be measured;

collecting a dissolved oxygen concentration of the water body measured $O_2^{sea,sensor}$ detected by the optical dissolved oxygen sensor in practical measurement; and

if the optical dissolved oxygen sensor in practical measurement has data drift, performing a calibration process including:

closing the water inlet and the gas outlet of the sealed cylinder, opening the water outlet and the gas inlet of the sealed cylinder, controlling the nitrogen bottle to inject and fill the cylinder body with nitrogen and drain out the water body measured;

collecting a dissolved oxygen concentration $O_2^{0\%air,sensor}$ detected by the optical dissolved oxygen sensor in practical measurement; and

calibrating the dissolved oxygen concentration $O_2^{sea,sensor}$ with a calibration model to obtain a calibration value of dissolved oxygen concentration $O_2^{corr}$ :

$$O_2^{corr} = D + E \times O_2^{sea,sensor},$$

in which,

$$D = -E \times O_2^{0\%air,sensor};$$

$$E = Y_2 / \left(O_2^{100\%air,sensor} - O_2^{0\%air,sensor}\right);$$

$$Y_2 = \left(1 - \frac{Y_1}{100}\right) O_2^{100\%air,sensor}.$$

Compared to the prior art, the advantages and positive effects of the present invention are as follows: the present invention uses gas calibration instead of solution calibration to create two pure gaseous environments of oxygen-free and saturated air for the optical dissolved oxygen sensor. The value of dissolved oxygen, temperature, pressure and humidity parameters in the two gaseous environments, combined with the constructed error model and calibration model, can not only determine whether the optical dissolved oxygen sensor has data drift problem, but also automatically calibrate to achieve accurate detection of the dissolved oxygen concentration in the measured medium. Since a pure gas environment can keep the oxygen concentration stable, the correction value obtained by the gas calibration method is more accurate than the solution calibration method. In addition, the calibration method uses saturated air and high-purity nitrogen as calibration gases, and it is not necessary to calibrate a 100% oxygen-saturated solution and oxygen-fee water. The procedure is therefore simpler than with solution calibration, and the calibration gas even diffuses into the air and does not pollute the atmosphere, and is clean and environmentally friendly.

Further features and advantages of the present invention will become clearer upon reading the detailed description of embodiments of the present invention in conjunction with the accompanying drawings.

Description of drawings

Fig.1 is a schematic diagram of the general structure of a calibration apparatus for a optical dissolved oxygen sensor according to the present invention;

Fig.2 is a flow chart of an error model setting phase in a calibration method for a optical dissolved oxygen sensor according to the present invention;

Fig.3 is a flow chart of a calibration phase in a calibration method for a optical dissolved oxygen sensor according to the present invention.

Detailed Description

Specific embodiments of the present invention are described in detail below with reference to the accompanying drawings.

It should be noted that in the description of the present invention, the expressions "top", "bottom", "inside", "outside" and other expressions indicating a direction or positional relationship are based on the direction or positional relationship shown in the drawings, are for convenience of description only and do not indicate or imply that the apparatus or element must have a particular orientation, be constructed in a particular orientation or function in a particular orientation, and therefore should not be construed as limiting the invention.

It should also be noted that in the description of the present invention, the terms "incorporated", "connected" and "attached" are to be understood in a broad sense, unless expressly stated and limited otherwise. For example, it may be a fixed connection, a detachable connection or an integral connection; it may be a mechanical connection or an electrical connection; it may be a direct connection or an indirect connection via an intermediate medium; and it may be the internal communication between the two components. For those skilled in the art, the specific meanings of the above terms in the present invention may be understood according to the specific situations.

The present embodiment is based on the rule that the optical dissolved oxygen sensor has different response characteristics in gaseous and liquid media, but a calibration model constructed in the gaseous environment is also applicable to the liquid environment, in which, gaseous calibration is used to replace solution calibration to establish an error model and a calibration model to automatically detect if the optical dissolved oxygen sensor has data drift and to also perform automatic calibration of the drifted measurement data.

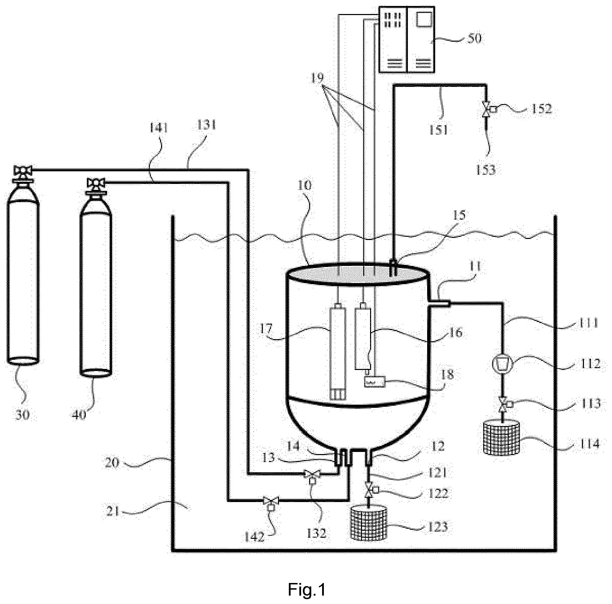

To achieve an in-situ calibration of an optical dissolved oxygen sensor when it is used, the present embodiment first proposes a structural design of an optical dissolved oxygen sensor calibration apparatus as shown in Fig.1, which comprises main components including a sealed cylinder 10, a constant temperature water tank 20, an air bottle 30, a data acquisition and control unit 50, etc.

The sealed cylinder 10 is preferably made of a material having fast heat conduction and good sealing properties, such as stainless steel. The sealed cylinder 10 is provided with a water inlet 11, a water outlet 12, gas inlets 13 and 14, a gas outlet 15 and the like.

The water inlet 11 serves to inject a water body to be measured into a cylinder body of the sealed cylinder 10 and is preferably arranged at the side wall of the cylinder body and near the top of the sealed cylinder 10, so that the sealed cylinder 10 can be conveniently filled with the water body to be measured. In this embodiment, the water inlet 11 is connected to a water inlet pipe 111 and a filter apparatus 114 is installed at the end of the water inlet pipe 111. Impurities in the water body to be measured are filtered out by the filter apparatus 114 before it enters the water inlet pipe 111. A water inlet solenoid valve 113 and a water circulation pump 112 can also be installed in the water inlet pipe 111 to enable automatic control of a water inlet process.

The water outlet 12 is used for draining the liquid from the cylinder body and is preferably arranged at the bottom of the sealed cylinder 10 to facilitate draining of all the liquid. Preferably, the bottom of the sealed cylinder 10 may be bowl-shaped and the water outlet 12 is located near the center of the bottom of the bowl so that the water body to be measured in the cylinder body can be drained cleanly and the residual can be minimized for reducing its impact on accuracy of the in-situ calibration. In this embodiment, the water outlet 12 is connected to a water outlet pipe 121, and a water outlet solenoid valve 122 may be arranged in the water outlet pipe 121 to implement automatic control of a water outlet process. A filtering device 123 may be installed at the end of the water outlet pipe 121, and the filtering device 123 may be used to filter impurities in the measured water body after use and then discharge them to the outside, thereby preventing contamination of the outside environment.

The gas inlets 13 and 14 are for introducing gas into the cylinder body and are preferably located at the bottom of the sealed cylinder 10. In this embodiment, two gas inlets 13 and 14 are preferably provided, one gas inlet 13 being connected to the air bottle 30 via an air pipe 131 for introducing saturated air into the cylinder body and the other gas inlet 14 being connected to the nitrogen bottle 40 via a nitrogen pipe 141 for introducing high purity nitrogen into the cylinder body. Other high purity gases except oxygen may of course be used instead of nitrogen for a calibration process of the optical dissolved oxygen sensor. An air solenoid valve 132 and a nitrogen solenoid valve 142 are preferably installed in the air pipe 131 and in the nitrogen pipe 141, respectively, to enable automatic control of a gas inlet process.

The gas outlet 15 is used to vent the cylinder body and is preferably arranged at the top of the sealed cylinder 10 and connected to an external gas outlet pipe 151. A gas outlet solenoid valve 152 can be arranged on the gas outlet pipe 151 to implement automatic control of a gas outlet process.

The cylinder body of the sealed cylinder 10 is allowed to accommodate an optical dissolved oxygen sensor 16 and a sensor module for detecting parameters such as temperature, pressure and humidity in the cylinder body, which includes a CTD temperature and salinity sensor 17, a humidity sensor 18 and the like as an example. The CTD temperature and salinity sensor 17 is configured to measure the temperature and the gas pressure (air pressure) inside the cylinder body and the humidity sensor 18 is configured to measure the gas humidity therein. Cables 19 connecting the optical dissolved oxygen sensor 16 to the sensor module are let out of the cylinder body and connected to the data acquisition and control unit 50 to transmit concentration of dissolved oxygen, temperature, pressure, humidity and other measurement data collected to the data acquisition and control unit 50.

The sealed cylinder 10 is placed in the constant temperature water tank 20, the constant temperature water tank 20 contains pure water 21, and the pure water 21 completely floods the sealed cylinder 10. The constant temperature water tank 20 allows the temperature in the sealed cylinder 10 to be controlled by changing the temperature of the pure water 21. To ensure the tightness of the sealed cylinder 10, a watertight part can be provided at the top of the sealed cylinder 10 and the cables 19 can be passed through the watertight part to prevent pure water 21 from entering the cylinder body through openings for the cables. The other openings of the sealed cylinder 10 (e.g. water inlet 11, water outlet 12, gas inlets 13, 14 and gas outlet 15 etc.) can be sealed with rubber gaskets.

The data acquisition and control unit 50 is able to control the switching states of the air bottle 30, a nitrogen bottle 40 and the water circulation pump 112 and the solenoid valves 113, 122, 132, 142, 152, so that the apparatus is able to perform the water inlet process, the water outlet process, the gas inlet process and the gas outlet process automatically.

In the following, an optical dissolved oxygen sensor calibration method of this embodiment is described in detail with reference to the optical dissolved oxygen sensor calibration apparatus shown in Fig.1.

The calibration method for the optical dissolved oxygen sensor in this embodiment comprises two phases: an error model setting phase and a calibration phase. The error model setting phase can be performed in the laboratory to generate a mathematical expression of the error model; the calibration phase can be performed in situ in practical measurement processes to meet the requirements for long-term and continuous monitoring of dissolved oxygen levels in seawater in the field of ocean observation.

(1) The error model setting phase

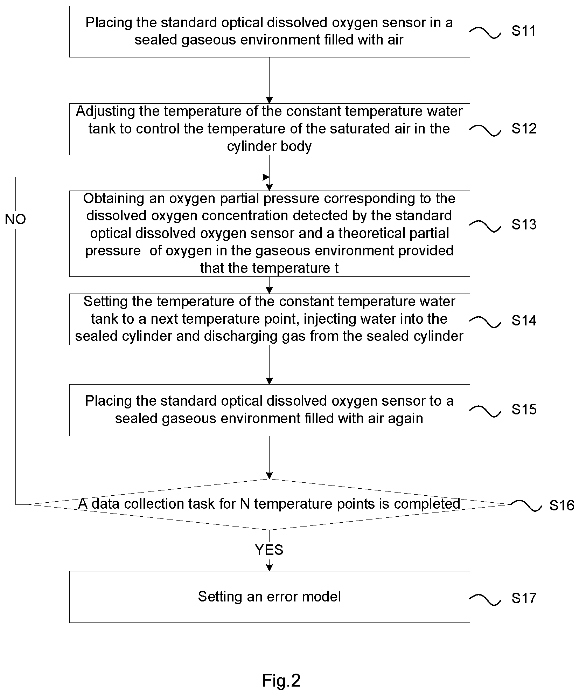

In this phase, the optical dissolved oxygen sensor calibration apparatus can be set up in the laboratory and a standard dissolved oxygen sensor (accurate, drift-free optical dissolved oxygen sensor) is installed in the sealed cylinder 10, and the accurate optical dissolved oxygen sensor is used to generate an error model. The phase is illustrated in Fig.2 and comprising the following steps.

S11. The standard optical dissolved oxygen sensor is placed in an air-filled sealed gaseous environment.

In this embodiment, the following method may be used to obtain the air-filled sealed gaseous environment.

The data acquisition and control unit 50 is used to open the water inlet solenoid valve 113 and the gas outlet solenoid valve 152, the other solenoid valves are closed, and the water circulation pump 112 is started to inject water into the sealed cylinder 10 until the cylinder body is filled with water so that the gas in the cylinder body is completely drained from an exhaust port 153 through the gas outlet pipe 151; then the water inlet solenoid valve 113, the air outlet solenoid valve 152 and the water circulation pump 112 are closed.

The air bottle 30, the air solenoid valve 132 and the water outlet solenoid valve 122 are opened to inject saturated air into the sealed cylinder 10. The saturated air entering the sealed cylinder 10 allows the water contained in the cylinder body to escape through the water outlet pipe 121. After all the water has been drained, the injection of air continues for a period of time before the air bottle 30, the air solenoid valve 132 and the water outlet solenoid valve 122 are closed. At this point, the cylinder body is filled with saturated air, so the standard optical dissolved oxygen sensor is in an air-filled sealed gaseous environment.

S12, adjusting the temperature of the constant temperature water tank 20 to control the temperature of the saturated air $t$ in the cylinder body.

In this embodiment, the objective of adjusting the saturated air temperature $t$ in the cylinder body can be achieved by adjusting the temperature of the pure water 21 in the constant temperature water tank 20.

In particular, a temperature range can be determined according to a range of the annual temperature change of the water body to be measured. For example, if the water body to be measured is seawater, a suitable temperature range may be determined according to a range of the annual temperature change at the location of the seawater to be measured. Within this temperature range, a sufficient number of temperature points (e.g. *N* temperature points) are then selected in a gradient fashion and the constant temperature water tank 20 is controlled so that the temperature of the pure water 21 in the tank is adjusted starting from the lowest temperature point within the temperature range.

S13, obtaining an oxygen partial pressure $O_2^{sensor}(t)$ corresponding to the concentration of dissolved oxygen detected by the standard optical dissolved oxygen sensor and a theoretical partial pressure of oxygen $O_2^{air}(t)$ in the air-filled gaseous environment provided that the temperature *t*.

In this embodiment, the temperature *t*, the pressure and the humidity of the saturated air in the cylinder body may be detected by the CTD temperature and salinity sensor 17 and humidity sensor 18 installed in the sealed cylinder 10, and the dissolved oxygen concentration of the saturated air in the cylinder body may be detected by the standard optical dissolved oxygen sensor. In a preferred embodiment, if the range of variation of the temperature detected by the CTD temperature and salinity meter 17 is within ±0.01 °C, and the range of variation of the dissolved oxygen concentration detected by the standard optical dissolved oxygen sensor is within ±0. 01 μmol/L, it is considered that the gasous environment in the cylinder body reaches a stable state, and the data acquisition and control unit 50 records the collected data including: the temperature *t*(°C) of the saturated air in the sealed cylinder 10, the air pressure *Air(t)* (i.e., the air pressure of the air-filled sealed gaseous environment in hector-Pascal, hPa), the humidity *RH*, and the dissolved oxygen concentration under the temperature *t*°C. A corresponding oxygen partial pressure $O_2^{sensor}(t)$ is calculated according to the dissolved oxygen concentration, and the unit is hPa.

When the temperature is *t*°C, the theoretical partial pressure value of oxygen $O_2^{air}(t)$ in saturated air in the sealed cylinder 10 (i.e. the theoretical partial pressure value of oxygen in the air-filled sealed gaseous environment, unit hPa) is calculated according to the following formula:

$$O_2^{air}(t) = \left[ Air(t) - \varnothing_{H_2O}(t) \times \left( \frac{RH}{100} \right) \right] \times M_{O_2} \qquad \text{(Formula 1)}$$

$$\varnothing_{H_2O}(t) = EXP(52.57 - 6690.9/T - 4.681 \times lnT) \qquad \text{(Formula 2)}$$

In the formula, *T* is the thermodynamic temperature of temperature *t* and the unit is Kelvin *K*, i.e. *T* = *t* + 273.15°C; *EXP* stands for the exponential function; $\frac{RH}{100}$ is the relative humidity of the saturated air in the sealed cylinder 10; $M_{O_2}$ is the mole fraction of oxygen.

S14, regulating the temperature of the constant temperature water tank 20 to a next temperature point, injecting water into the sealed cylinder 10, and discharging gas from the sealed cylinder 10.

In this embodiment, the water inlet solenoid valve 113, the gas outlet solenoid valve 152 and the water circulation pump 112 may be opened when the temperature of the pure water 21 in the constant temperature water tank 20 reaches the next temperature point, so as to inject water into the sealed cylinder 10 to remove the saturated air in the cylinder body through the exhaust port 153 until the cylinder body is filled with water and the saturated air in the cylinder body is completely removed, the water outlet solenoid valve 122 can be opened to circulate the water in the cylinder body for a period of time, and then the water inlet solenoid valve 113, the gas outlet solenoid valve 152 and the water circulation pump 112 are closed.

S15, placing the standard optical dissolved oxygen sensor to the air-filled sealed gaseous environment again.

The air bottle 30, the air solenoid valve 132 and the water outlet solenoid valve 122 are opened and saturated air is again injected into the sealed cylinder 10. The saturated air entering the sealed cylinder 10 displaces the water contained in the cylinder body, which is drained through the water outlet pipe 121. After all the water has been discharged, the injection of air continues for a period of time before the air bottle 30, the air solenoid valve 132 and the water outlet solenoid valve 122 are closed. At this point, the cylinder body is filled with saturated air, so the standard optical dissolved oxygen sensor is in the air-filled sealed gaseous environment.

S16, executing the S13 and repeating the process until a data collection task for *N* temperature points is completed.

At this point, *N* values of $O_2^{sensor}(t)$ and *N* values of $O_2^{air}(t)$ are collected.

S17, setting an error model.

In this embodiment, the error model established is

$$Y_1(t) = At^2 + Bt + C \qquad\qquad \text{(Formula 3)}$$

Wherein *A, B,* and *C* are constant coefficients, $Y_1(t) = \dfrac{O_2^{sensor}(t) - O_2^{air}(t)}{O_2^{air}(t)}\%$ . The values of the coefficients *A, B,* and *C* can be determined according to different temperature points *t* and $Y_1(t)$.

The specific process is:

Inputting *N* temperature points *ti* and *N* values of $Y_1(ti)$ to the error model $Y_1(t) = At^2 + Bt + C$, and calculating multiple groups of *A, B* and *C* values (usually *N* - 3 groups of *A, B,* and *C* values).

Fitting the obtained multiple *A* values, multiple *B* values and multiple *C* values, respectively, to determine final values of the *A, B,* and *C* coefficients. In this embodiment, *N* is preferably an integer greater than or equal to 10; the greater *N* is, the more accurate the determined error model is.

After the error model is established, it is stored in the data acquisition and control unit 50.

(2) Calibration phase

Calibration of the concentration data of dissolved oxygen detected by the optical dissolved oxygen sensor may be carried out in the laboratory or on site at the actual monitoring location.

If the measurement medium is seawater, the optical dissolved oxygen sensor calibration apparatus is preferably installed on an autonomous marine observation platform. While monitoring the dissolved oxygen concentration of seawater, the optical dissolved oxygen sensor can be monitored periodically to automatically assess whether the sensor has a data drift, and the dissolved oxygen concentration (measurement data) detected by the optical dissolved oxygen sensor with a data drift problem is automatically calibrated to ensure the accuracy of the monitoring results.

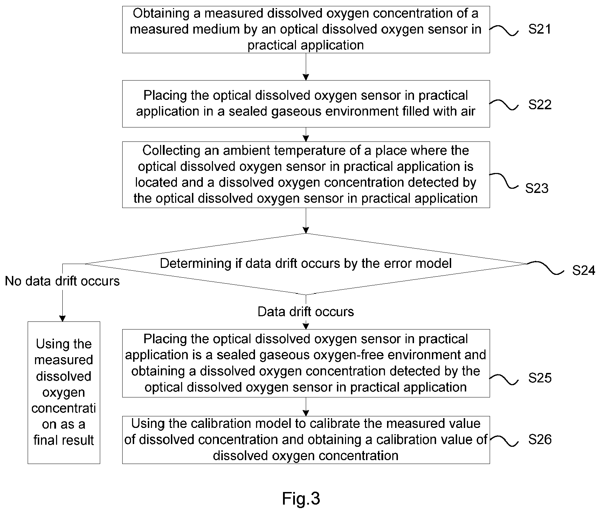

The calibration phase disclosed by the present embodiment is a process of calibrating a measured dissolved oxygen concentration $O_2^{sea,sensor}$ of a measured medium detected by an optical dissolved oxygen sensor in practical measurement by the calibration model. The process is shown in Fig.3 and includes:

S21, obtaining a measured dissolved oxygen concentration $O_2^{sea,sensor}$ of a measured medium by an optical dissolved oxygen sensor in practical measurement.

If the measured medium is seawater, an optical dissolved oxygen sensor in practical measurement is placed in the sealed cylinder 10, and the entire optical dissolved oxygen sensor calibration apparatus can be mounted on the autonomous marine observation platform to perform in-situ measurement and calibration of the dissolved oxygen concentration of seawater.

In this process, the water inlet solenoid valve 113, the water outlet solenoid valve 122 and the water circulation pump 112 can first be opened, the seawater can be in-situ sucked by the water circulation pump 112 in which the impurities contained therein can be filtered out by the filter apparatus 114, and then flows into the sealed cylinder 10 through the water inlet pipe 111; a portion of the seawater in the cylinder body is returned to the sea through the water outlet pipe 121 and the filter device 123, thereby creating a seawater circulation. After the cylinder body is filled with seawater, all the solenoid valves and the water circulation pump 112 are closed, and the data acquisition and control unit 50 collects the dissolved oxygen concentration detected by the optical dissolved oxygen sensor 16 in the sealed cylinder 10 to obtain the measured dissolved oxygen concentration $O_2^{sea,sensor}$ .

S22, placing the optical dissolved oxygen sensor in practical measurement in an air-filled sealed gaseous environment.

In this process, the air solenoid valve 132, the water outlet solenoid valve 122 and the air bottle 30 may be opened and saturated air is injected into the sealed cylinder 10 through the air pipe 131. The seawater in the sealed cylinder 10 is forced out of the sealed cylinder 10 by the saturated air entering the cylinder body and is returned to the sea through the water outlet pipe 121. After the seawater in the cylinder body is completely drained, the injection of saturated air continues for a period of time to prevent the remaining seawater in the cylinder body affecting the assessment result of whether the optical dissolved oxygen sensor 16 has a problem of data drift. Thereafter, the air solenoid valve 132, the water outlet solenoid valve 122 and the air bottle 30 are closed to create a fully sealed space in the sealed cylinder 10, i.e., the optical dissolved oxygen sensor 16 is placed in a sealed gaseous environment that is filled with air.

S23, collecting an ambient temperature $t0$ of a place where the optical dissolved oxygen sensor in practical measurement is located and a dissolved oxygen concentration $O_2^{100\%air,sensor}$ detected by the optical dissolved oxygen sensor in practical measurement.

In this embodiment, after the gaseous environment in the sealed cylinder 10 is stabilized, the data acquisition and control unit 50 collects the temperature $t0$, the air pressure $Air(t0)$, the humidity $RH$ detected by the CTD temperature and salinity sensor 17 and the dissolved oxygen concentration $O_2^{100\%air,sensor}$ detected by the optical dissolved oxygen sensor 16 in practical measurement in the cylinder body, and converts the dissolved oxygen concentration $O_2^{100\%air,sensor}$ to a corresponding oxygen partial pressure value $O_2^{sensor}(t0)$。

S24. Using the error model to determine if data drift occurs in the optical dissolved oxygen sensor in practical measurement; if no data drift occurs, the measured dissolved oxygen concentration $O_2^{sea,sensor}$ is directly used as a result; if data drift occurs, the following calibration process is executed.

In this embodiment, the ambient temperature $t0$ in the cylinder body is substituted into the error model (formula 3) to calculate $Y_1$, namely

$Y_1 = Y_1(t0) = At0^2 + Bt0 + C$ ; in the meanwhile, using the formula 1 and formula 2 to calculate $\frac{O_2^{sensor}(t0)-O_2^{air}(t0)}{O_2^{air}(t0)}\%$

; then calculating the difference between $Y_1$ and $\frac{O_2^{sensor}(t0)-O_2^{air}(t0)}{O_2^{air}(t0)}\%$ ; wherein $O_2^{air}(t0)$ is a theoretical partial pressure value of oxygen in the saturated air in the sealed cylinder 10 at temperature $t0$ and may be calculated by Formula 1 and Formula 2.

If the difference is within a set error range (for example, an error range $\pm5\%$), it is determined that the optical dissolved oxygen sensor 16 in practical measurement has no data drift; on the contrary, if the difference is beyond the set error range, it is determined that the optical dissolved oxygen sensor 16 in practical measurement has data drift.

S25. Placing the optical dissolved oxygen sensor in practical measurement in an oxygen-free sealed gaseous environment and obtaining a dissolved oxygen concentration $O_2^{0\%air,sensor}$ detected by the optical dissolved oxygen sensor in practical measurement.

In this embodiment, the water inlet solenoid valve 113, the gas outlet solenoid valve 152 and the water circulation pump 112 can be opened first and seawater is injected into the sealed cylinder 10. The seawater in the cylinder body is used to remove the saturated air in the sealed cylinder 10 through the gas outlet pipe 151. After the cylinder body is filled with seawater, the gas outlet solenoid valve 152 is closed and the water outlet solenoid valve 122 is opened to allow the seawater in the cylinder body to circulate for a period of time, then the water inlet solenoid valve 113 and the water circulation pump 112 are closed, and the nitrogen valve 142 and the nitrogen bottle 40 are opened to inject nitrogen into the sealed cylinder 10 through the nitrogen pipe 141. The nitrogen entering the cylinder body pushes the seawater in the cylinder body to flow back to the sea through the water outlet pipe 121. After the seawater in the cylinder body has been completely drained, the nitrogen continues to be injected for a period of time before the water outlet solenoid valve 122, the nitrogen solenoid valve 142 and the nitrogen bottle 40 are closed. At this point, the sealed cylinder 10 is filled with high purity nitrogen so that the optical dissolved oxygen sensor 16 in practical measurement is placed in the oxygen-free sealed gaseous environment.

After the gaseous environment in the sealed cylinder 10 is stabilized, the data acquisition and control unit 50 collects the dissolved oxygen concentration $O_2^{0\%air,sensor}$ detected by the optical dissolved oxygen sensor in practical measurement.

S26. Using the calibration model to calibrate the measured value of dissolved oxygen concentration $O_2^{sea,sensor}$ and obtaining a calibration value of dissolved oxygen concentration $O_2^{corr}$.

In this embodiment, a calibration model in the following could be used to calibrate the $O_2^{sea,sensor}$ :

$$O_2^{corr} = D + E \times O_2^{sea,sensor},$$

in which,

$$D = -E \times O_2^{0\%air,sensor};$$

$$E = Y_2 / \left( O_2^{100\%air,sensor} - O_2^{0\%air,sensor} \right);$$

$$Y_2 = \left(1 - \frac{Y_1}{100}\right) O_2^{100\%air,sensor};$$

in these formula, the unit of each parameter representing the dissolved oxygen concentration is $\mu$mol/L.

Up to this point a calibration process of the dissolved oxygen concentration is measured medium is finished.

The optical dissolved oxygen calibration method and apparatus in this embodiment can be used on various autonomous observation platforms to realize long-term, continuous and automatic monitoring of the dissolved oxygen content in the measured medium so that the obtained measurement data can be continuously and accurately maintained, and is particularly suitable for the field of marine environmental monitoring.

The foregoing, of course, represents only one preferred embodiment of the present invention. It should be noted that those skilled in the art will be able to make various improvements and modifications without departing from the principles of the present invention. Improvements and modifications are also to be considered within the scope of the present invention.

**Claims**

1. An optical dissolved oxygen sensor calibration method, **characterized in that** it comprises:

generating an error model with an accurate optical dissolved oxygen sensor, which includes:

placing a standard optical dissolved oxygen sensor in an air-filled sealed gaseous environment;

under each of different temperatures $t$, obtaining an oxygen partial pressure value $O_2^{sensor}(t)$ corresponding to a dissolved oxygen concentration detected by the standard optical dissolved oxygen sensor and a theoretical partial pressure value of oxygen $O_2^{air}(t)$ in the air-filled sealed gaseous environment; and establishing an error model: $Y_1(t) = At^2 + Bt + C$, wherein $A$, $B$, and $C$ are coefficients and determined by

$$Y_1(t) = \frac{O_2^{sensor}(t) - O_2^{air}(t)}{O_2^{air}(t)} \%$$

and the temperature $t$;

and,

calibrating a measured dissolved oxygen concentration $O_2^{sea,sensor}$ of a measured medium detected by an optical dissolved oxygen sensor in practical measurement by a calibration model, which includes:

placing the optical dissolved oxygen sensor in practical measurement in an air-filled sealed gaseous environment to obtain a dissolved oxygen concentration $O_2^{100\%air,sensor}$ detected by the optical dissolved oxygen sensor in practical measurement;

placing the optical dissolved oxygen sensor in practical measurement in an oxygen-free sealed gaseous environment and obtaining a dissolved oxygen concentration $O_2^{0\%air,sensor}$ detected by the optical dissolved oxygen sensor in practical measurement; and

using a calibration model to calibrate the measured dissolved oxygen concentration $O_2^{sea,sensor}$ to obtain a calibration value of dissolved oxygen concentration $O_2^{corr}$; in which the calibration model is:

$$O_2^{corr} = D + E \times O_2^{sea,sensor},$$

in which,

$$D = -E \times O_2^{0\%air,sensor};$$

$$E = Y_2 / \left( O_2^{100\%air,sensor} - O_2^{0\%air,sensor} \right);$$

$$Y_2 = \left( 1 - \frac{Y_1}{100} \right) O_2^{100\%air,sensor};$$

$$Y_1 = Y_1(t0) = At0^2 + Bt0 + C ;$$

$t0$ is the temperature of the measured medium.

2. The optical dissolved oxygen sensor calibration method according to claim 1, **characterized in that**, a method to determine the values of the coefficients *A, B,* and *C* including:

   selecting *N* temperature points *ti* in a determined temperature range, *i* = 1,2, ......,*N*;
   calculating *N* values of $Y_1$(*ti*) corresponding to the *N* temperature points *ti*:

$$Y_1(ti) = \frac{O_2^{sensor}(ti) - O_2^{air}(ti)}{O_2^{air}(ti)} \%;$$

   inputting N temperature points *ti* and N values of $Y_1$(*ti*) to the error model $Y_1(t) = At^2 + Bt + C$, and calculating multiple groups of *A, B* and *C* values; and
   fitting the obtained multiple *A* values, multiple *B* values and multiple *C* values, respectively, to determine final values of the *A, B,* and *C* coefficients.

3. The optical dissolved oxygen sensor calibration method according to claim 2, **characterized in that**, the temperature range is determined according to a range of an annual temperature change of the medium to be measured by the optical dissolved oxygen sensor in practical measurement, and $N \geq 10$.

4. The optical dissolved oxygen sensor calibration method according to claim 1, **characterized in that**, the theoretical partial pressure value of oxygen $O_2^{air}(t)$ is calculated by:

$$O_2^{air}(t) = \left[ Air(t) - \emptyset_{H_2O}(t) \times \left( \frac{RH}{100} \right) \right] \times M_{O_2}$$

$$\emptyset_{H_2O}(t) = EXP(52.57 - 6690.9/T - 4.681 \times lnT)$$

   wherein *T* is the thermodynamic temperature of temperature *t* and the unit is Kelvin *K*; the unit of *t* is °C; $\frac{RH}{100}$ is the relative humidity of the air-filled sealed gaseous environment; $M_{O_2}$ is the mole fraction of oxygen; and *Air(t)* is the air pressure of the air-filled sealed gaseous environment under the temperature *t*.

5. The optical dissolved oxygen sensor calibration method according to any one of claim 1 to claim 4, **characterized in that** :

the air-filled sealed gaseous environment is created by filling a sealed cylinder with saturated air; and
the oxygen-free sealed gaseous environment is created by filling the sealed cylinder with nitrogen.

6. An optical dissolved oxygen sensor calibration apparatus, **characterized in that** it includes:

a sealed cylinder including a cylinder body provided with a water inlet, a water outlet, gas inlets and a gas outlet, which is configured to accommodate an optical dissolved oxygen sensor;
a constant temperature water tank containing liquids flooding the sealed cylinder;
an air bottle connected to one gas inlet of the sealed cylinder via an air pipe, which is configured to inject saturated air into the cylinder body of the sealed cylinder;
a nitrogen bottle connected to the other gas inlet of the sealed cylinder via a nitrogen pipe, which is configured to inject nitrogen into the cylinder body of the sealed cylinder;
a sensor module disposed in the cylinder body of the sealed cylinder, which is configured to detect the temperature, pressure and humidity inside the cylinder body; and
a data acquisition and control unit configured to execute a data acquisition and control process including:

controlling the oxygen bottle to inject air into the sealed cylinder to fill the cylinder body with saturated air;
collecting a temperature inside the cylinder body $t0$ detected by the sensor module and a dissolved oxygen concentration $O_2^{100\%air,sensor}$ detected by the optical dissolved oxygen sensor in practical measurement accommodated in the cylinder body; calculating an oxygen partial pressure value $O_2^{sensor}(t)$ corresponding to the dissolved oxygen concentration;
calculating $Y_1(t)$, wherein $Y_1 = Y_1(t0) = At0^2 + Bt0 + C$; wherein $A$, $B$, and $C$ are constant coefficients;
calculating a difference between $Y_1$ and $\frac{O_2^{sensor}(t0)-O_2^{air}(t0)}{O_2^{air}(t0)}\%$; determining that the optical dissolved oxygen sensor in practical measurement has data drift if the difference is beyond a set error range; wherein $O_2^{air}(t0)$ is a theoretical partial pressure value of oxygen in the saturated air in the sealed cylinder under the temperature $t0$;
opening the water inlet and the gas outlet of the sealed cylinder, injecting and filling the cylinder body with a water body to be measured;
collecting a dissolved oxygen concentration of the water body measured $O_2^{sea,sensor}$ detected by the optical dissolved oxygen sensor in practical measurement; and
if the optical dissolved oxygen sensor in practical measurement has data drift, performing a calibration process including:

closing the water inlet and the gas outlet of the sealed cylinder, opening the water outlet and the gas inlet of the sealed cylinder, controlling the nitrogen bottle to inject and fill the cylinder body with nitrogen and drain out the water body measured;
collecting a dissolved oxygen concentration $O_2^{0\%air,sensor}$ detected by the optical dissolved oxygen sensor in practical measurement; and
calibrating the dissolved oxygen concentration $O_2^{sea,sensor}$ with a calibration model to obtain a calibration value of dissolved oxygen concentration $O_2^{corr}$:

$$O_2^{corr} = D + E \times O_2^{sea,sensor},$$

in which,

$$D = -E \times O_2^{0\%air,sensor};$$

$$E = Y_2/\left(O_2^{100\%air,sensor} - O_2^{0\%air,sensor}\right);$$

$$Y_2 = \left(1 - \frac{Y_1}{100}\right) O_2^{100\%air,sensor}.$$

7. The optical dissolved oxygen sensor calibration apparatus according to claim 6, **characterized in that** the data acquisition and control unit executes another data acquisition and control process in a setting error model phase including:

controlling the oxygen bottle to inject air into and fill the sealed cylinder with saturated air;
adjusting the temperature of the constant temperature water tank and collecting the temperature *t,* the air pressure *Air(t)*, the humidity *RH* inside the cylinder body detected by the sensor module and the oxygen partial pressure $O_2^{sensor}(t)$ corresponding to the dissolved oxygen concentration detected by the optical dissolved oxygen sensor in practical measurement accommodated in the sealed cylinder under different temperature conditions;
setting an error model $Y_1(t) = At^2 + Bt + C$; determining the values of coefficients *A, B,* and *C* according to

$$Y_1(t) = \frac{O_2^{sensor}(t) - O_2^{air}(t)}{O_2^{air}(t)}\%$$ and

the temperature *t*;
wherein $O_2^{air}(t)$ is a theoretical partial pressure value of oxygen in saturated air in the sealed cylinder under the temperature *t* and calculated by:

$$O_2^{air}(t) = \left[Air(t) - \emptyset_{H_2O}(t) \times \left(\frac{RH}{100}\right)\right] \times M_{O_2}$$

$$\emptyset_{H_2O}(t) = EXP(52.57 - 6690.9/T - 4.681 \times lnT)$$

wherein *T* is the thermodynamic temperature of temperature *t* and the unit is Kelvin *K;* the unit of *t* is °C; $\frac{RH}{100}$ is the relative humidity of the air-filled sealed gaseous environment; $M_{O_2}$ is the mole fraction of oxygen.

8. The optical dissolved oxygen sensor calibration apparatus according to claim 7, **characterized in that** the data acquisition and control unit is configured to determine the values of coefficients *A, B,* and *C* by:

selecting *N* temperature points according to a set temperature gradient in a determined temperature range which is obtained according to a range of the annual temperature change of the water body to be measured;
adjusting the temperature of the constant temperature water tank and stabilizing the temperature in sequence at the *N* temperature points;
collecting the temperature of the saturated air *ti* at each temperature point, *i* = 1,2, ... ..., *N*;
$$Y_1(ti) = \frac{O_2^{sensor}(ti) - O_2^{air}(ti)}{O_2^{air}(ti)}\%$$
calculating N values of $Y_1(ti)$ by ;
inputting *N* temperature points *ti* and *N* values of $Y_1(ti)$ to the error model $Y_1(t) = At^2 + Bt + C$ and calculating multiple groups of *A, B* and *C* values; and
fitting the obtained multiple *A* values, multiple *B* values and multiple *C* values, respectively, to determine final values of the *A, B,* and *C* coefficients.

9. The optical dissolved oxygen sensor calibration apparatus according to claim 6, **characterized in that** the data acquisition and control unit performs a water inlet process before controlling the oxygen bottle to inject air into the sealed cylinder, during which first filling water into the sealed cylinder, then opening the gas inlet and the water outlet, controlling the oxygen bottle to inject air into the sealed cylinder to use the saturated air entering into to drain out the water body measured in the cylinder body, and then closing the gas inlet and the water outlet to keep the cylinder body filled with saturated air.

**10.** The optical dissolved oxygen sensor calibration apparatus according to any one of claim 6 to claim 9, **characterized in that**:

the water inlet is arranged at the side wall of the cylinder body and near the top of the sealed cylinder and connected to a water inlet pipe onto which a filter apparatus, a water inlet solenoid valve and a water circulation pump are arranged;

the water outlet is arranged at the bottom of the sealed cylinder and connected to a water outlet pipe onto which a filter device and a water outlet solenoid valve are arranged;

the gas outlet is arranged at the top of the sealed cylinder and connected to a gas outlet pipe onto which a gas outlet solenoid valve is arranged;

the gas inlets are arranged at the bottom of the sealed cylinder wherein:

an air inlet connected to an air pipe onto which an air solenoid valve is arranged; and

a nitrogen inlet connected to a nitrogen pipe onto which a nitrogen solenoid valve is arranged;

wherein the data acquisition and control unit automatically perform a water inlet process, a water outlet process, a gas inlet process and a gas outlet process automatically by controlling switching states of the solenoid valves and the water circulation pump.

Fig.1

Placing the standard optical dissolved oxygen sensor in a sealed gaseous environment filled with air — S11

Adjusting the temperature of the constant temperature water tank to control the temperature of the saturated air in the cylinder body — S12

Obtaining an oxygen partial pressure corresponding to the dissolved oxygen concentration detected by the standard optical dissolved oxygen sensor and a theoretical partial pressure of oxygen in the gaseous environment provided that the temperature t — S13

Setting the temperature of the constant temperature water tank to a next temperature point, injecting water into the sealed cylinder and discharging gas from the sealed cylinder — S14

Placing the standard optical dissolved oxygen sensor to a sealed gaseous environment filled with air again — S15

NO

A data collection task for N temperature points is completed — S16

YES

Setting an error model — S17

Fig.2

Obtaining a measured dissolved oxygen concentration of a measured medium by an optical dissolved oxygen sensor in practical application ⌒ S21

↓

Placing the optical dissolved oxygen sensor in practical application in a sealed gaseous environment filled with air ⌒ S22

↓

Collecting an ambient temperature of a place where the optical dissolved oxygen sensor in practical application is located and a dissolved oxygen concentration detected by the optical dissolved oxygen sensor in practical application ⌒ S23

↓

Determining if data drift occurs by the error model ⌒ S24

No data drift occurs ← → Data drift occurs

Using the measured dissolved oxygen concentration as a final result

Placing the optical dissolved oxygen sensor in practical application is a sealed gaseous oxygen-free environment and obtaining a dissolved oxygen concentration detected by the optical dissolved oxygen sensor in practical application ⌒ S25

↓

Using the calibration model to calibrate the measured value of dissolved concentration and obtaining a calibration value of dissolved oxygen concentration ⌒ S26

Fig.3

## INTERNATIONAL SEARCH REPORT

International application No.

**PCT/CN2021/128294**

### A. CLASSIFICATION OF SUBJECT MATTER

G01N 21/64(2006.01)i

According to International Patent Classification (IPC) or to both national classification and IPC

### B. FIELDS SEARCHED

Minimum documentation searched (classification system followed by classification symbols)

G01N

Documentation searched other than minimum documentation to the extent that such documents are included in the fields searched

Electronic data base consulted during the international search (name of data base and, where practicable, search terms used)

CNTXT, CNKI, WPI, EPODOC: 山东省科学院海洋仪器仪表研究所, 张颖颖, 溶解氧, 传感, 校正, 校准, 漂移, 温度, 误差, 模型, dissolved, oxygen, DO, sensor, water, sea, temperature, correct+, error, model

### C. DOCUMENTS CONSIDERED TO BE RELEVANT

| Category* | Citation of document, with indication, where appropriate, of the relevant passages | Relevant to claim No. |
|---|---|---|
| A | CN 108680551 A (INSTITUTE OF OCEANOGRAPHIC INSTRUMENTATION, SHANDONG ACADEMY OF SCIENCES) 19 October 2018 (2018-10-19) description, paragraphs [0004]-[0017] | 1-10 |
| A | CN 108663347 A (INSTITUTE OF OCEANOGRAPHIC INSTRUMENTATION, SHANDONG ACADEMY OF SCIENCES) 16 October 2018 (2018-10-16) entire document | 1-10 |
| A | CN 111089845 A (SUZHOU FUYANG ENVIRONMENTAL PROTECTION TECHNOLOGY CO., LTD.) 01 May 2020 (2020-05-01) entire document | 1-10 |
| A | JP S5963567 A (TOSHIBA K. K.) 11 April 1984 (1984-04-11) entire document | 1-10 |
| A | CN 105973954 A (LIHERO TECHNOLOGY (HUNAN) CO., LTD.) 28 September 2016 (2016-09-28) entire document | 1-10 |
| A | CN 113358732 A (AI-SENSING TECHNOLOGY (GUANGDONG) CO., LTD.) 07 September 2021 (2021-09-07) entire document | 1-10 |

☐ Further documents are listed in the continuation of Box C. ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **12 April 2022** | **26 April 2022** |

| Name and mailing address of the ISA/CN | Authorized officer |
|---|---|
| China National Intellectual Property Administration (ISA/CN) No. 6, Xitucheng Road, Jimenqiao, Haidian District, Beijing 100088, China | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/128294**

| Patent document cited in search report | | | Publication date (day/month/year) | Patent family member(s) | | | Publication date (day/month/year) |
|---|---|---|---|---|---|---|---|
| CN | 108680551 | A | 19 October 2018 | None | | | |
| CN | 108663347 | A | 16 October 2018 | None | | | |
| CN | 111089845 | A | 01 May 2020 | None | | | |
| JP | S5963567 | A | 11 April 1984 | JP | H0464026 | B2 | 13 October 1992 |
| CN | 105973954 | A | 28 September 2016 | None | | | |
| CN | 113358732 | A | 07 September 2021 | None | | | |

Form PCT/ISA/210 (patent family annex) (January 2015)